# EUROPÄISCHE PATENTSCHRIFT

(11) **EP 0 807 404 B1**
(45) Veröffentlichungstag und Bekanntmachung des Hinweises auf die Patenterteilung: **26.03.2003**
(21) Anmeldenummer: 97107307.7
(22) Anmeldetag: 02.05.1997
(51) Int. Cl.: A61B 6/02

(54) **Röntgendiagnostikgerät für Tomosynthese**
Diagnostic x-ray apparatus for tomosynthesis
Appareil de radiodiagnostique tomosynthétique

(30) Priorität: 17.05.1996 DE 19619925
(43) Veröffentlichungstag der Anmeldung: 19.11.1997
(73) Patentinhaber: Sirona Dental Systems GmbH, 64625 Bensheim (DE)
(72) Erfinder: Plötz, Josef, Dr., 64625 Bensheim (DE); Franetzki, Manfred, Dr., 64625 Bensheim (DE)
(74) Vertreter: Sommer, Peter

(56) Entgegenhaltungen:
- US-A- 4 211 927
- US-A- 4 907 251

## Beschreibung

Aus der WO 93/22 893 A1 ist eine Methode bekannt, mit der es möglich ist, eine Aufnahme eines Untersuchungsobjektes zu rekonstruieren, ohne das hierbei die Projektionswinkel α und die geometrische Anordnung von Strahlensender und Strahlenempfänger und die Fokalebene bekannt sind. Gemäß dieser Methode ist im Bereich des Strahlenempfängers eine Referenz aus strahlenabsorbierendem Material mit bekannter Größe und bekanntem Abstand zum Strahlenempfänger vorgesehen, die bei jeder Einzelprojektion auf den Strahlenempfänger projiziert wird. Aufgrund der örtlichen Abbildung der Referenz auf den Strahlenempfänger für jede Einzelprojektion kann die geometrische Anordnung und der zweidimensionale Projektionswinkel α ermittelt werden. Das Referenzobjekt ist hierbei an einem Aufbißhalter angeordnet, der auch den Strahlenempfänger trägt.

Eine Halterung zum Positionieren eines Strahlensenders eines Röntgendiagnostikgeräts für Tomosynthese ist aus der DE 44 14 689 A1 bekannt. An die Halterung ist ein Tragarm angekoppelt, an dem - in Strahlungsrichtung gesehen - vor dem Untersuchungsobjekt ein kugelförmiges Referenzobjekt und hinter dem Untersuchungsobjekt ein Strahlenempfänger angeordnet sind. Über die Halterung wird zum einen der Abstand des Strahlensenders zum Referenzobjekt und zum Strahlenempfänger sowie der Winkel α eines vom Strahlensender emittierten Strahlenbündels zu einer Bezugsachse der Haltevorrichtung vorgegeben. Es ist ferner bekannt, die Strahlenquelle verstellbar in einem Gehäuse anzuordnen, an das eine Positionierungsvorrichtung für das Referenzobjekt und den Strahlenempfänger ankoppelbar ist.

Bei der Tomosynthese müssen für alle der mehreren Projektionen (Einstrahlwinkel) die relativen Positionen und Lagen von Strahlensender, Untersuchungsobjekt und Strahlenempfänger bekannt sein. Wie bereits erläutert wird hierzu gemäß der WO 93/22 893 eine Auswertung von Bildsignalen vorgenommen, die beim Durchstrahlen des Referenzobjektes, das starr mit dem Strahlenempfänger verbunden ist, erzeugt werden. Hierbei ergibt sich beim Durchstrahlen des Untersuchungsobjektes allerdings eine unerwünschte Überlagerung durch das Referenzobjekt und es bedarf eines hohen Verarbeitungsaufwandes um die durch das Referenzobjekt verursachte Überlagerung aus den Bildsignalen heraus zu rechnen.

Gemäß der DE 44 14 689 A1 werden diese hierzu notwendigen Informationen durch die Konstruktion, d.h. durch die fest vorgegebenen Anordnung von Strahlensender, Untersuchungsobjekt und Strahlenempfänger vorgegeben. Aufgrund des hohen konstruktiven Aufwandes sind die Kosten eines derart ausgeführten Röntgendiagnostikgerätes erhöht. Zudem ist eine starre Fixierung des Untersuchungsobjektes gegeben was von diesem als unangenehm empfunden wird.

Als nachteilig wird bei beiden Lösungsvorschlägen angesehen, daß Veränderungen der Lagebeziehung zwischen dem Aufnahmeobjekt und dem Strahlenempfänger nicht erfaßt werden.

Aufgabe der Erfindung ist daher die weitere vorteilhafte Ausgestaltung des Röntgendiagnostikgerätes der eingangs genannten Art.

Die Aufgabe wird erfindungsgemäß durch den Gegenstand des Patentanspruches 1 gelöst.

Vorteil der Erfindung ist die Verwendung von Positionsdetektoren zum Erfassen der Zuordnung von Strahlensender zum Strahlenempfänger oder zum Untersuchungsobjekt, so daß keine mechanisch starre Kopplung vorgesehen werden muß oder ein Referenzobjekt sich durch Überlagerung störend auswirkt.

Es ist vorteilhaft, wenn keine Kopplung, eine Kopplung zum Begrenzen der relativen Verstellung, oder eine elastische Kopplung zwischen dem Strahlensender und dem Strahlenempfänger oder dem Untersuchungsobjekt besteht, so daß auch dem Untersuchungsobjekt eine gewisse Bewegungsfreiheit erlaubt ist. Besonders vorteilhaft ist eine Kopplung, bei der bei einer geringen Abweichung von der Soll-Zuordnung ein kleines Rückstellmoment und bei einer großen Abweichung ein großes Rückstellmoment wirkt.

Werden in Abhängigkeit von der Zuordnung des Strahlensenders zum Strahlenempfänger oder zum Untersuchungsobjekt Korrektursignale erzeugt, so ist es vorteilhaft, den Strahlensender zur Emission von Strahlung nur dann anregen zu können, wenn vorgegebene Grenzen der Korrektursignale nicht überschritten werden oder eine Anzeige erfolgt, wenn die Korrektursignale die vorgegebenen Grenzen überschreiten. Es wird somit sichergestellt, daß verwacklungsfreie Aufnahmen erhalten werden, wodurch die Strahlenbelastung durch wiederholtes Durchstrahlen des Untersuchungsobjektes zum Erhalt einwandfreier Aufnahmen reduziert ist.

Weitere Vorteile und Einzelheiten der Erfindung ergeben sich aus der nachfolgenden Beschreibung eines Ausführungsbeispieles anhand der Zeichnung in Verbindung mit den Unteransprüchen.

Es zeigt:
- FIG 1: Ein Röntgendiagnostikgerät für Tomosynthese in prinzipieller Darstellung,
- FIG 2: ein Blockschaltbild des Röntgendiagnostikgerätes nach FIG 1,
- FIG 3: ein Ausführungsbeispiel zum Erfassen der Bewegung des Untersuchungsobjektes relativ zum Strahlenempfänger,
- FIG 4, FIG 5: ein am Untersuchungsobjekt angeordneter Positionsdetektor,
- FIG 6: eine Vorrichtung mit Positionsdetektoren nach der FIG 5 in einer Draufsicht und
- FIG 7: eine Koppeleinrichtung des Röntgendiagnostikgeräts nach FIG 1.

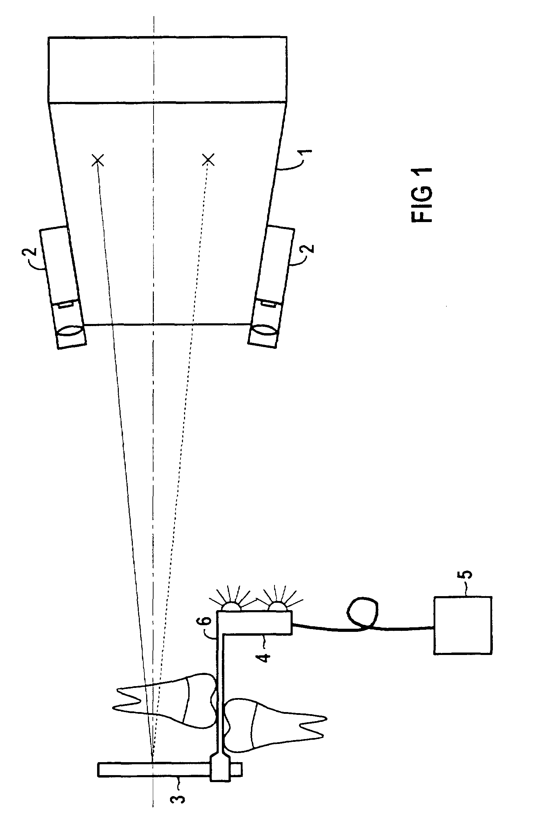

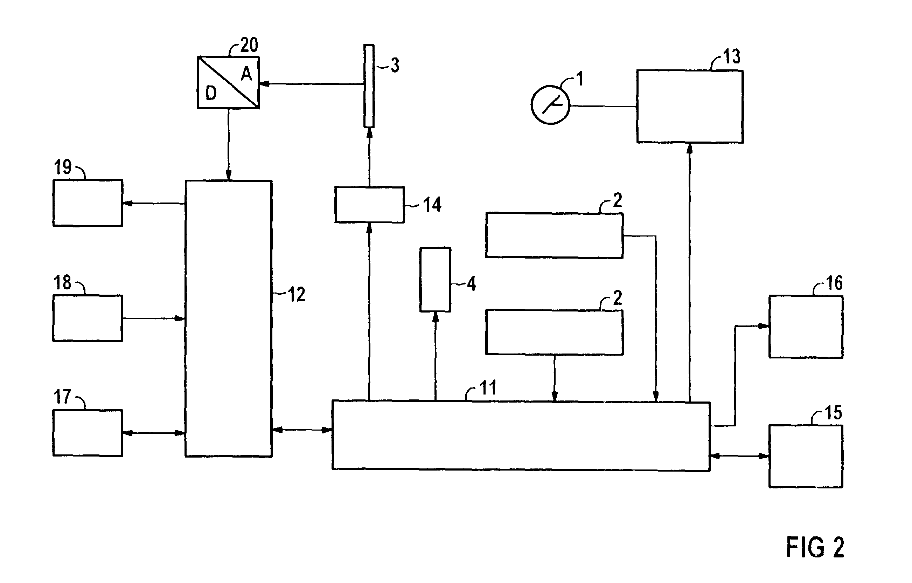

In der FIG 1 ist ein Röntgendiagnostikgerät für Tomosynthese dargestellt, das einen Strahlensender 1 aufweist, dem im Ausführungsbeispiel ein Empfänger 2 eines Positionsdetektors zugeordnet ist. Dem Strahlensender 1 ist ein Strahlenempfänger 3 zugeordnet, der die vom Strahlensender 1 ausgehende Strahlung empfängt. Diesem Strahlenempfänger 3 ist ein Sender 4 des Positionsdetektors zugeordnet. Im Ausführungsbeispiel ist der Sender 4 als Infrarotsender ausgeführt, dessen Licht vom Empfänger 2 in elektrische Signale gewandelt wird. Diese elektrischen Signale werden einer Auswerteeinrichtung 11 (FIG 2) zugeführt, die hieraus den Abstand, den Einstrahlwinkel und die Einstrahlrichtung bestimmt. Dem Sender 4 wird die Spannung einer Spannungsquelle 5 zugeführt. Im Ausführungsbeispiel sind sowohl der Strahlenempfänger 3 als auch der Sender 4 an einer als Aufbißhalter ausgeführten Halterung 6 angeordnet.

Im Rahmen der Erfindung kann der Positionsdetektor als magnetische, elektromagnetische, optische, akustische oder mechanische Einrichtung ausgeführt sein. Ferner kann ein Sender 4 des Positionsdetektors am Strahlensender 1 und ein Empfänger 2 des Positionsdetektors dem Strahlenempfänger 3 zugeordnet sein. Bei einer weiteren Ausgestaltung ist beispielsweise der Sender 4 am Strahlensender 1 vorgesehen und sendet ein Strahlenbündel, das von einer dem Strahlenempfänger 3 zugeordneten Referenzstelle reflektiert und von einem am Strahlensender 1 vorgesehenen Empfänger 2 empfangen wird.

Zwischen dem Strahlensender 1 und dem Strahlenempfänger 3 kann, wie in der FIG 1 gezeigt, keine Kopplung bestehen. Es ist aber auch möglich, eine Kopplung zur Begrenzung der relativen Verstellung oder eine elastische Kopplung derart vorzusehen, daß dem Untersuchungsobjekt ein gewisser Bewegungsspielraum eingeräumt wird. Besonders vorteilhaft ist es, wenn die Kopplung so ausgeführt ist, daß bei einer geringen Abweichung von einer Soll-Zuordnung ein kleines Rückstellmoment und bei einer großen Abweichung ein großen Rückstellmoment erzeugt wird.

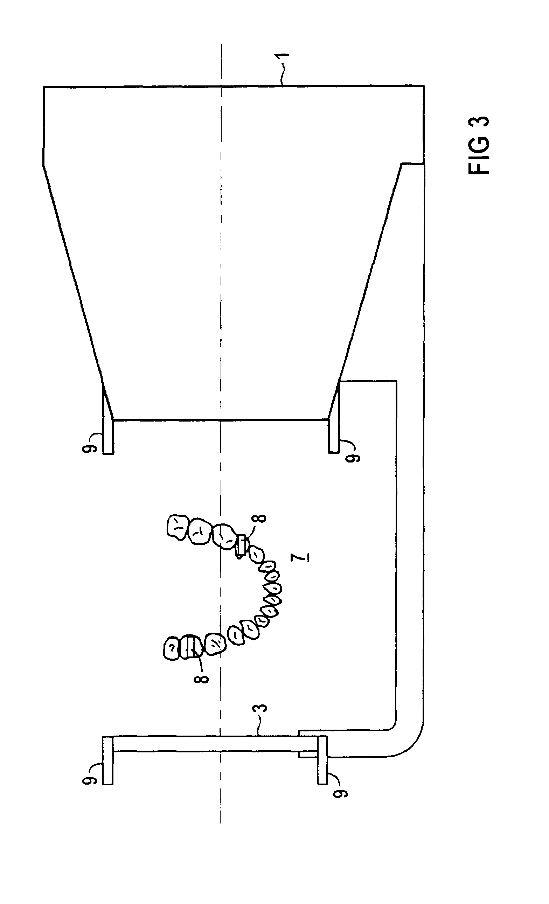

Aus den FIG 3, 4 und 5 geht hervor, daß der Positionsdetektor nicht nur, wie dies in der FIG 1 dargestellt ist, eine feste Zuordnung zum Strahlensender 3 haben, sondern er kann auch dem Untersuchungsobjekt direkt und unmittelbar zugeordnet sein. Aus der FIG 3 geht hervor, daß dem Untersuchungsobjekt 7, beispielsweise dem Kiefer, die Magnete 8, zugeordnet sind, deren Magnetfelder von Magnetfeldsensoren 9 am Strahlenempfänger 3 und/oder am Strahlensender 1 empfangen und in elektrische Signale gewandelt werden, die dann der Auswerteeinrichtung 11 zugeführt werden. Als Positionsdetektor kann auch eine elektromagnetische Einrichtung Anwendung finden, bei der entweder die Magnetfelder von Spulen erzeugt oder auch mittels Spulen detektiert werden können. Unter Verwendung von sich ändernden Magnetfeldern kann die Messung von statischen Störfeldern entkoppelt und die Genauigkeit der Messung verbessert werden.

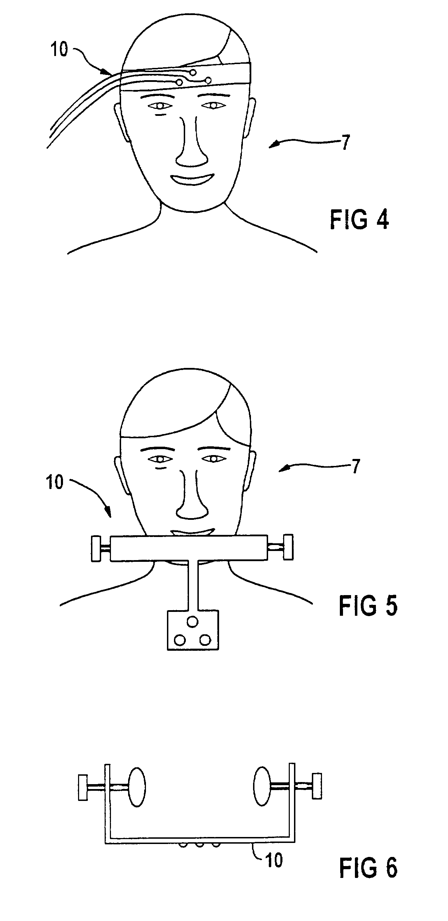

Der Sender oder Empfänger des Positionsdetektors kann auch am Untersuchungsobjekt 7, beispielsweise am Kopf, insbesondere an der Stirn (FIG 4), oder an einer Halteeinrichtung 10 am Kinn angeordnet sein. Durch die Anordnung des Positionsdetektors in möglichst großem Abstand zum Untersuchungsobjekt 7 kann die Meßgenauigkeit erheblich erhöht werden, weil beim Bewegen des Untersuchungsobjektes 7 aus der Ruhelage ein größerer Hebel, d.h. eine größere Auslenkung wirksam ist.

Eine erfindungsgemäße Röntgendiagnostikeinrichtung weist, wie sich aus der FIG 2 ergibt, neben dem Strahlensender 1, dem Positionsdetektor 2,4 und dem Strahlenempfänger 3 noch einen Steuerrechner 11 und einen Bildrekonstruktionsrechner 12 auf. Der Steuerrechner 11 steuert eine Spannungsversorgungseinrichtung 13 für den Strahlensender 1, den Sender 4 des Positionsdetektors, eine Signalsteuereinrichtung 14 für den Strahlenempfänger 3 und hat eine Verbindung zum Bildrekonstruktionsrechner 12 zu einem Speicher 15 und zu einer Anzeige 16 und empfängt Signale vom Empfänger 2 des Positionsdetektors. Dem Bildrekonstruktionsrechner 12 ist ein weiterer Speicher 17, Bedienelemente 18, eine Ausgabeeinheit 19 sowie ein Analog-/Digital-Wandler zugeordnet, der die vom Strahlenempfänger 3 ausgehenden Signale wandelt. Der Steuerrechner 11 berechnet, wie bereits erläutert, aus den Signalen des Positionsdetektors den Abstand des Strahlensenders 1 zum Strahlenempfänger 3, den Einstrahlwinkel und die Einstrahlrichtung. Weicht die Ausrichtung von Strahlensender 1, Untersuchungsobjekt 7 und Strahlenempfänger 3 von einer Soll-Zuordung ab, so bildet der Steuerrechner 11 Korrektursignale, die entweder sofort oder zu einem späteren Zeitpunkt, nach vorübergehender Speicherung im Speicher 15, dem Bildrekonstruktionsrechner 12 zugeführt werden und die bei der Berechnung eines Durchstrahlungsbildes des Untersuchungsobjektes 7 berücksichtigt werden. Es ist vorteilhaft, wenn in Abhängigkeit von den Korrektursignalen der Strahlensender 1 zur Emission von Strahlung anregbar ist, wenn vorgegebene Grenzen der Korrektursignale nicht überschritten werden. Es kann auch über die Anzeige 16 eine Anzeige erfolgen, durch die mitgeteilt wird, daß beispielsweise aufgrund der Bewegung des Untersuchungsobjektes 7 keine verwacklungsfreie Aufnahme erstellt werden kann.

Bei Panorama-Röntgengeräten zur Erstellung von Schichtaufnahmen wird das Untersuchungsobjekt 7 üblicherweise mittels Aufbißhalter, Stirnstütze oder Ohroliven möglichst starr am Röntgengerät fixiert, was als unangenehm empfunden wird. Eine Bewegung des Untersuchungsobjektes 7 führt allerdings zu einer Störung im erzeugten Röntgenbild, was ebenfalls unerwünscht ist. Auch hier ist es möglich, wie bereits erläutert, dem Untersuchungsobjekt 7 eine gewisse Bewegungsfreiheit zu geben und seine genaue Position und räumliche Orientierung und deren Änderung während des Aufnahmeablaufes durch Messung mittels eines Positionsdetektors zu erfassen. Aus den Signalen des Positionsdetektors werden dann Korrektursignale gebildet, um vor der Durchführung des tomographischen Verwischungsprozesses den Objektdetails aus der gewünschten, scharf abzubildenden Schicht, die richtigen Bildinhalte zuzuordnen und die Lage der gewünschten Schicht der Patientenbewegung nachzuführen. Voraussetzung hierfür ist, daß die entstehenden, sich entsprechend der tomographischen Abtastbewegung des Röntgengerätes laufend ändernden Bildsignale in feinen Schritten erfaßt und zunächst gespeichert werden und erst nach Vorliegen der kompletten für einen Objektbereich relevanten Bildsignale die Verwischung rechnerisch entsprechend der in diesem Objektbereich gewünschten Schichtlage zu erstellen. Die aus den Positionsmessungen erzeugten Korrektursignale beeinflussen dann die Ableitungsintervalle für die Einzelbilder/Einzelbildsignale sowie deren Verschiebung parallel und senkrecht zur tomographischen Abtastrichtung und beinhalten auch Rotationen der Einzelbilder/Einzelbildsignale, nach Richtung und Position der erstellten Schichtaufnahme differenzierte Änderungen des Vergrößerungsmaßstabes in den Einzelbildern/Einzelbildsignalen und das Zusammensetzen effektiver Einzelbilder/Einzelbildsignale aus mehreren Bildstreifen/Bildsignalstreifen gemäß in der Richtung senkrecht zur tomographischen Abtastrichtung variierender Ableitintervalle.

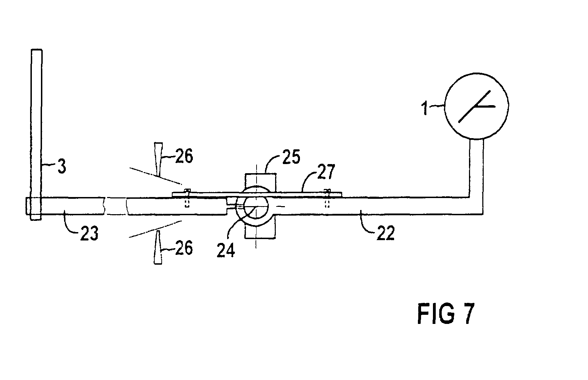

In der FIG 7 ist eine Koppeleinrichtung 21 in prinzipieller Weise dargestellt, die die relative Verstellung zwischen dem Strahlensender 1 und dem Strahlenempfänger 3 oder dem Untersuchungsobjekt 7 oder zwischen dem Strahlenempfänger 3 und dem Untersuchungsobjekt 7 begrenzt oder elastisch koppelt. Diese Koppeleinrichtung 21 weist einen ersten Arm 22 auf, der mit dem Strahlensender 1 in Verbindung steht. An einem zweiten Arm 23 der Koppeleinrichtung 21 ist der Strahlenempfänger 3 angeordnet. Das in der FIG 7 nicht gezeigte Untersuchungsobjekt ist üblicherweise im Bereich des Strahlenempfängers 3 angeordnet. Der erste und zweite Arm 22,23 sind um eine Drehachse 24 schwenkbar gelagert. Zur Erfassung der Verschwenkung der Arme 22,23 kann ein Wegaufnehmer 25 vorgesehen sein. Als Wegaufnehmer 25 eignen sich beispielsweise Rotationsencoder oder Dehnmeßstreifen. Der Koppeleinrichtung 21 sind zwei Wegbegrenzer 26 zugeordnet, die im Ausführungsbeispiel die Verstellbarkeit des zweiten Armes 23 begrenzen. Ist ein Federelement 27, beispielsweise eine Blattfeder vorgesehen, das mit den Armen 22,23 in Verbindung steht, so ist eine elastische Verstellung der Arme 22,23 relativ zueinander und um die Drehachse 24 möglich. Das Federelement 27 kann vorteilhaft so ausgeführt sein, daß es bei einer kleinen Abweichung von der gezeigten Soll-Zuordnung von Strahlensender 1 und Strahlenempfänger 3 zueinander ein kleines Rückstellmoment und bei einer großen Abweichung ein großes Rückstellmoment bewirkt. Die vom Wegaufnehmer 25 erzeugten Signale werden, wie bereits erläutert, der Auswerteeinrichtung 11 zugeführt. Im Rahmen der Erfindung können die Arme 22,23 auch noch um eine zur Drehachse 24 senkrechte Achse oder über ein kardanisches Drehgelenk in anderen Raumrichtungen verstellbar sein.

## Patentansprüche

1. Röntgendiagnostikgerät für Tomosynthese mit einen Strahlensender (1) und einem Strahlenempfänger (3), die zur Erstellung von Tomosyntheseaufnahmen eines Untersuchungsobjektes (7) verstellbar gelagert sind, so daß bei der Durchstrahlung des Untersuchungsobjektes (7) aus unterschiedlichen Richtungen am Strahlenempfänger (3) jeweils Signale ableitbar sind, die einer Bilderzeugungseinrichtung (12) zum Berechnen einer Tomosyntheseaufnahme des Untersuchungsobjektes (7) zugeführt werden,
mit zumindest einem Positionsdetektor (2,4) zum Erfassen der Zuordnung von Strahlensender (1) zum Strahlenempfänger (3) oder zum Untersuchungsobjekt (7) oder von Strahlenempfänger (3) zum Untersuchungsobjekt (7) zueinander und
mit einer Auswerteeinrichtung (11) zum Auswerten der Signale des Positionsdetektors (2,4),
wobei der Positionsdetektor (2,4) am Strahlensender (1), am Strahlenempfänger (3) oder am Untersuchungsobjekt (7) angeordnet ist
wobei Korrektursignale erzeugt werden, wenn die Zuordnung des Strahlensenders (1) zum Strahlenempfänger (3) oder zum Untersuchungsobjekt (7) oder vom Strahlenempfänger (3) zum Untersuchungsobjekt (7) von einer Soll-Zuordnung abweicht und
wobei die Korrektursignale der Bilderzeugungseinrichtung (12) zugeführt werden.

2. Röntgendiagnostikgerät nach Anspruch 1,
wobei der Positonsdetektor (2,4) als magnetische, elektromagnetiche, optische, akustische oder mechanische Einrichtung ausgeführt ist und
wobei der Sender- oder der Empfänger (2,4) des Postionsdetektors am Strahlenempfänger (3) oder am Untersuchungsobjekt (7) oder an einer mit diesem in Verbindung stehenden Halteeinrichtung (10) gelagert ist,
wobei der Empfänger oder der Sender (4, 2) am Strahlensender (1) angeordnet ist.

3. Röntgendiagnostikgerät nach Anspruch 1 oder 2,
wobei keine Kopplung, eine Kopplung zum Begrenzen der relativen Verstellung, oder eine elastische Kopplung zwischen dem Strahlensender (1) und dem Strahlenempfänger (3) oder dem Untersuchungsobjekt (7) besteht.

4. Röntgendiagnostikgerät nach Anspruch 3,
wobei die Kopplung derart ausgeführt ist, daß bei einer geringen Abweichung von der Soll-Zuordnung ein kleines Rückstellmoment und bei einer großen Abweichung ein großes Rückstellmoment wirkt.

5. Röntgendiagnostikgerät nach einem der Ansprüche 1 bis 4,
wobei in Abhängigkeit von den Korrektursignalen der Strahlensender (1) zu Emission von Strahlung anregbar ist, wenn vorgegebene Grenzen der Korrektursignale nicht überschritten werden.

6. Röntgendiagnostikgerät nach einem der Ansprüche 1 bis 5,
wobei eine Anzeige erfolgt, wenn die Korrektursignale die vorgegebenen Grenzen überschreiten.

## Claims

1. X-ray diagnostic apparatus for tomosynthesis, having a radiation transmitter (1) and a radiation receiver (3) which, for the generation of tomosynthesis recordings of an object (7) under examination, are adjustably mounted such that when the object (7) under examination is transilluminated from different directions, in each case signals can be derived at the radiation receiver (3) and are fed to an image-generating device (12) in order to calculate a tomosynthesis recording of the object (7) under examination,
having at least one position detector (2, 4) to register the relationship between the radiation transmitter (1) and the radiation receiver (3) or the object (7) under examination, or of radiation receiver (3) and object (7) under examination, and
having an evaluation device (11) to evaluate the signals from the position detector (2, 4),
the position detector (2, 4) being arranged on the radiation transmitter (1), on the radiation receiver (3) or on the object (7) under examination,
correction signals being generated if the relationship between the radiation transmitter (1) and the radiation receiver (3) or the object (7) under examination, or between the radiation receiver (3) and the object (7) under examination, deviates from a desired relationship and
the correction signals being fed to the image generating device (12).

2. X-ray diagnostic apparatus according to Claim 1, the position detector (2, 4) being implemented as a magnetic, electromagnetic, optical, acoustic or mechanical device and
the transmitter or the receiver (2, 4) of the position detector being mounted on the radiation receiver (3) or on the object (7) under examination or on a holding device (10) which is connected to the latter,
the receiver or the transmitter (4, 2) being arranged on the radiation transmitter (1).

3. X-ray diagnostic apparatus according to Claim 1 or 2, there being no coupling, coupling to limit the relative adjustment, or elastic coupling between the radiation transmitter (1) and the radiation receiver (3) or the object (7) under examination.

4. X-ray diagnostic apparatus according to Claim 3, the coupling being implemented such that a small restoring torque acts in the event of a small deviation from the desired relationship and a large restoring torque acts in the event of a large deviation.

5. X-ray diagnostic apparatus according to one of Claims 1 to 4, the radiation transmitter (1) being able to be stimulated, depending on the correction signals, to emit radiation if prescribed limits on the correction signals are not exceeded.

6. X-ray diagnostic apparatus according to one of Claims 1 to 5, an indication being given if the correction signals exceed the prescribed limits.

## Revendications

1. Appareil de diagnostic à rayons X pour tomosynthèse comportant
un émetteur de rayonnement (1) et un récepteur de rayonnement (3) qui sont montés de façon mobile en vue de réaliser des enregistrements pour tomosynthèse d'un objet d'études (7) de sorte que, lors de l'exposition à un rayonnement de l'objet d'études (7), des signaux peuvent provenir de différentes directions au niveau du récepteur de rayonnement (3), lesquels signaux sont envoyés à un dispositif de génération d'image (12) pour calculer un enregistrement de tomosynthèse de l'objet d'études (7),
au moins un détecteur de position (2, 4) pour déterminer la correspondance entre l'émetteur de rayonnement (1) et le récepteur de rayonnement (3) ou l'objet d'études (7) ou entre le récepteur de rayonnement (3) et l'objet d'études (7) et
un dispositif d'exploitation (11) destiné à exploiter les signaux du détecteur de position (2, 4),
le détecteur de position (2, 4) étant monté sur l'émetteur de rayonnement (1), sur le récepteur de rayonnement (3) ou sur l'objet d'études (7),
des signaux de correction étant générés lorsque la correspondance entre l'émetteur de rayonnement (1) et le récepteur de rayonnement (3) ou l'objet d'études (7) ou entre le récepteur de rayonnement (3) et l'objet d'études (7) s'écarte d'une consigne, et les signaux de correction étant envoyés au dispositif de génération d'image (12).

2. Appareil de diagnostic à rayons X selon la revendication 1, dans lequel le détecteur de position (2, 4) est conformé en dispositif magnétique, électromagnétique, optique, acoustique ou mécanique, et dans lequel
l'émetteur ou le récepteur (2, 4) du détecteur de position est monté sur le récepteur de rayonnement (3) ou sur l'objet d'études (7) ou sur un dispositif de support (10) relié à celui-ci,
le récepteur ou l'émetteur (4, 2) étant monté sur l'émetteur de rayonnement (1).

3. Appareil de diagnostic à rayons X selon la revendication 1 ou 2, dans lequel il n'est pas prévu de couplage ou il est prévu un couplage destiné à limiter le déplacement relatif, ou un couplage élastique entre l'émetteur de rayonnement (1) et le récepteur de rayonnement (3) ou l'objet d'études (7).

4. Appareil de diagnostic à rayons X selon la revendication 3, dans lequel le couplage est conformé de façon à générer un petit couple de rappel dans le cas d'un petit écart à la consigne et un grand couple de rappel important dans le cas d'un grand écart.

5. Appareil de diagnostic selon l'une des revendications 1 à 4, dans lequel l'émetteur de rayonnement (1) destiné à émettre un rayonnement peut être excité en fonction des signaux de correction, lorsque des limites prédéterminées des signaux de correction ne sont pas dépassées.

6. Appareil de diagnostic à rayons X selon la l'une des revendications 1 à 5, dans lequel il est effectué un affichage lorsque les signaux de correction dépasse des limites prédéterminées.
